# EUROPEAN PATENT APPLICATION

(11) **EP 2 913 064 A1**
(43) Date of publication of application: **02.09.2015**
(21) Application number: 14156844.4
(22) Date of filing: 26.02.2014
(51) Int. Cl.: A61K 47/48, A61P 35/00

(54) **Branched drug-linker conjugates for the coupling to biological targeting molecules**

(71) Applicant: celares GmbH, 13125 Berlin (DE)
(72) Inventor: Baranowski, Matthias, 16552 Schildow (DE); Leenders, Frank, 13187 Berlin (DE); Krähmer, Ralf, 16341 Panketal (DE)
(74) Representative: Andrae | Westendorp Patentanwälte Partnerschaft

(57) **Abstract**

The invention relates to novel conjugates comprising three molecules of a therapeutic agent and a branched, mono-reactive polyethylene glycol (PEG) based linker according to formula (I) or (II), a method for the preparation of such conjugates and use thereof. In addition, the invention relates to a pharmaceutical composition, a pharmaceutical kit comprising said conjugates for the treatment of a disease or disorder that can be at least in part alleviated by therapy.

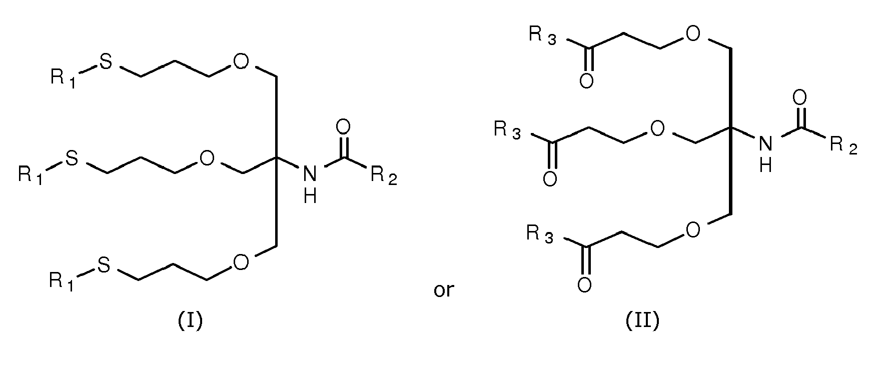

## Description

### SUMMARY

The present invention relates to novel conjugates comprising three molecules of a therapeutic agent and a branched, mono-reactive polyethylene glycol (PEG) based linker, a method for the preparation of such conjugates and use thereof. In addition, the invention relates to a pharmaceutical composition, a pharmaceutical kit comprising said conjugates for the treatment of a disease or disorder that can be at least in part alleviated by therapy.

These novel conjugates are ideally suited for the covalent coupling to biological targeting molecules, e.g. being from the group of proteins, peptides or oligonucleotides. Conjugates of the present invention facilitate the coupling of three therapeutic agents to a single attachment site of a biological targeting molecule. In this way, the targeting properties of the biological molecule are maintained. At the same time, the payload with the therapeutic agent can be maximised without involving multiple attachment sites. However, in case of multiple attachment sites of the biological targeting molecule a strong increase of the payload can be achieved. The resulting molecule combines the properties of the biological targeting molecule with those of the therapeutic agent. Thus, conjugates of the present invention can be used to produce therapeutic drugs with beneficial pharmacological properties that are very specific and show high therapeutic effect for a target tissue, respectively.

Synthesis of conjugates of the present invention is performed in organic solvents. This is particularly advantageous because many therapeutic agents are hydrophobic and poorly soluble in aqueous solutions. After coupling of the therapeutic agent to the PEG based branched mono-activated linker according to this invention, the resulting conjugates are very good soluble in both organic solvents and aqueous solutions. This amphiphilic property facilitates a coupling of these conjugates to biological molecules under conditions that prevent their inactivation or aggregation, preferably in aqueous buffers systems. In this way, the present invention makes the manufacturing process for conjugates of therapeutic drugs and biological molecules very mild and more compatible for the latter.

### BACKGROUND

Monoclonal antibodies (mMabs, used synonymously with the terms antibody or immuno-globuline in the following) have led to the development of approved biopharmaceutical products in many therapeutic areas, including cardiovascular diseases, infection, immune disorders and cancer. Examples are Rituxan or Herceptin (Trastuzumab) that have successfully been used for the treatment of cancer.

Antibodies are characterised by their high specificity for particular target molecules. However, due to their high molecular weights of 150 kilo Dalton or more, their penetration to some tissues and in this way their applicability is limited. Furthermore, the formation of resistance towards a therapy with immunoglobulines has been reported for the treatment of HER2 positive breast cancer with Herceptin (Trastuzumab). Thus, with few exceptions, the curative effect of immunoglobulines in human therapy fell far short of expectations. For this reason, large efforts have been made in the past years to load tissue specific antibodies with high potential cytotoxic drugs.

Therapeutic agents and in particular cytotoxic agents often display little selectivity for the target tissue. Consequently, high doses of the cytotoxic agents are required to achieve a therapeutic effect. However, this proceeding goes along with strong side effects because of the substantial toxicity that cytotoxic agents exhibit to normal tissues. Thus, the coupling of a cytotoxic drug to an antibody (antibody-drug conjugate (ADC)) has been investigated intensively aiming to reduce systemic toxicity and increasing the therapeutic benefit for patients. This basic principle of coupling cytotoxic drugs to receptor binding proteins has already been published at the late nineties of the last century or even earlier.

At present, several novel antibody-drug conjugates are under development or have already been approved such as antibody conjugates with Maytansinoide, Taxane or Doxorubicin. Numerous targets on tumour or endothelial cells as well as in the subendothelial extracellular matrix have been identified to be attractive for ADC development.

Synthesis of ADCs is achieved by coupling the cytotoxic agent to amino acid residues, disulfide bridges or sugar residues of the antibody via a bi-functional cross-linker. Specificity of the coupling reaction can be achieved by introducing rare or unnatural amino-acid residues to the sequence of the antibody. However, still the predominant ways of coupling are the modification of the ε-amino group of lysine residues or the thiol group of a free cysteine. In any case, the synthesis of ADCs typically proceeds in two steps. 1. Coupling of the bi-functional crosslinker to either the antibody or to the cytotoxic agent and 2. Reacting the resulting conjugate with the antibody or vice versa.

This proceeding requires bi-functional cross-linkers with two divergent reactivities. Typically, one is a thiol specific reactivity such as maleimide, pyridyldisulfide or iodo acetamide. The other reactivity facilitates a modification of amino- or carboxyl groups. Free thiol groups are very rare and thus often have to be introduced post-translational to one of the reactants. This can be achieved for example by using additional linkers bearing a thiol or disulphide group, reduction of disulfide bridges in the antibody or the introduction of thiol groups using Traut's reagent. The cytotoxic agent of the resulting conjugate is then probably released lysosomally causing death of the respective cell.

Early bi-functional cross-linkers used for the production of ADCs were short hydrophobic pH labile linkers. Mylotarg (Gemtuzumab ozogamicin) was the first approved ADC bearing a pH sensitive 4-(4'-acetylphenoxy) butanoic acid linker. It allows for attachment to surface-exposed lysines of the antibody over an amide bond and forms an acyl hydrazone linkage with N-actetyl-γ-calicheaminicin dimethyl hydrazide. Upon internalisation of the ADC, the calicheamicin prodrug is released by hydrolysis of the hydrazone in lysosomes. However, Mylotarg had limited success probably because the linker was not sufficiently stable and released too much of the drug to the bloodstream.

Second generation bi-functional cross-linkers were more stable and allowed a sensitive adjustment of pH stability, redox stability, or of resistance towards enzymatic cleavage. However, one common feature of these linkers was their hydrophobicity. For example, the commercially available cross-linkers succinimidyl 4-(N-maleimidomethyl)cyclohexane-1-carboxylate (SMCC) and N-succinimidyl 3-(2-pyridyldithio) propionate (SPDP) are hydrophobic. Typically, ADCs reported in the art based on such hydrophobic cross-linkers contain no more than 3-4 drug molecules per antibody molecule. Attempts to attain higher drug/antibody ratios often failed, particularly if both the cytotoxic agent and the linker were hydrophobic, because of ADC aggregation, loss of affinity for the target antigen, and rapid clearance from circulation.

However, conjugation with high drug/antibody ratios could be beneficial in situations where the target antigen density on the cell surface is low and offers a means of boosting potency. Thus, recent research focussed on the development of water soluble bi-functional cross-linkers. Zhao et al. (Zhao R.Y. et al. (2011), "Synthesis and Evaluation of Hydrophilic linkers for Antibody-Maytansinoid Conjugates", J.Med.Chem. 54, 3606-3623) synthesised and evaluated hydrophilic linkers for antibody-Maytansinoid conjugates (AMCs). They reported that incorporation of water soluble non-reducible linkers into AMCs provided two benefits. First, the ability to deliver a higher concentration of Maytansinoid into the target cells per antibody binding event using AMCs with high drug antibody ration is made possible by using hydrophilic linkers. Second, AMCs with hydrophilic linkers generate metabolites that may be poorly effluxed out of the tumor cells by MDR transporters, resulting in greater activity toward multi-drug resistant cells.

The principle of conjugating small therapeutic agents to biological targeting molecules is not limited to antibodies. Other molecules have been reported to be very efficient in the delivery of such agent, such as human serum albumin, transferrin, fibrinogen or folic acid.

In conclusion, the use of water soluble linkers for ADCs and conjugates of therapeutic agents with biological targeting molecules appears to be very promising to improve their properties. However, current approaches use linear PEG linkers that allow the attachment of only one therapeutic agent per site. Thus, there is a great need for drug-linker molecules that
- Show excellent solubility in aqueous solution,
- Introduce high payloads of therapeutic agent per attachment site,
- Minimise loss of activity of the biological targeting molecule, and
- Prevent aggregation of the biological targeting molecule to be modified.

### DETAILED DESCRIPTION OF THE INVENTION

It is an object of the invention to provide conjugates comprising three molecules of a therapeutic agent and a branched, mono-reactive linker suitable for the covalent coupling to biological targeting molecules being selected from the group consisting of proteins, peptides, carbohydrates or oligonucleotides. Examples for useful targeting molecules are antibodies, antibody fragments, scaffold proteins, receptor binding proteins, lectins, haptenes or serum albumin, transferrin, fibrinogen or folic acid.

It is a further object to provide conjugates that are water soluble and thus can be coupled to the biological targeting molecule in aqueous solutions. In this way, inactivation and aggregation of the biological targeting molecule can be prevented.

A further object of the invention is to provide a method for the preparation of conjugates comprising three molecules of a therapeutic agent and a branched, mono-reactive linker in organic solvents. This is particularly advantageous because many therapeutic agents are hydrophobic and poorly soluble in aqueous solutions. After coupling of the therapeutic agent to the PEG based branched mono-activated the resulting conjugate is very good soluble in both organic solvents and aqueous solutions.

It is a further object of the invention to provide functionalities that facilitate the coupling of conjugates comprising three molecules of a therapeutic agent and a branched, mono-reactive linker to reactive groups of a targeting molecule. These functionalities facilitate the binding to a biological targeting molecule selected from the group consisting of proteins, peptides, carbohydrates or oligonucleotides, in particular antibodies, antibody fragments, scaffold proteins, serum proteins, receptor binding proteins, nanobodies, lectins, haptenes, polymers and natural products within one reaction step.

Due to the fact that the conjugates according to the invention comprise three therapeutic agents per conjugate, the payload per attachment site of the biological targeting molecule is at least 3 resulting in superior properties as far as the efficacy is concerned.

These objects of the present invention are solved by conjugates of the following formulas: 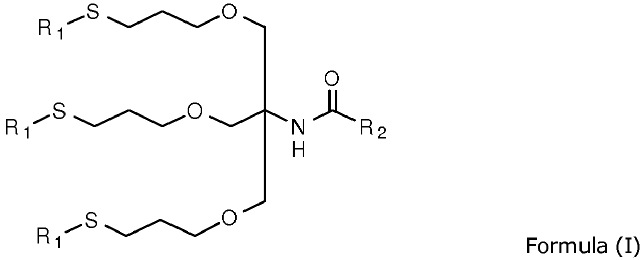 and 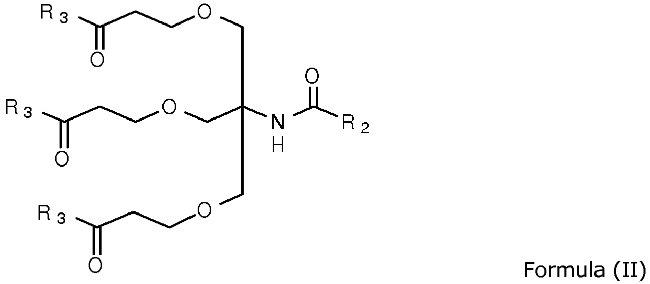 wherein R₁ represents

D-X-(CH₂)ₘ Formula (III)

or

D-X-(CH₂-CH₂-O)ₙ-(CH₂)ₘ Formula (IV)

or

D-X-(CH₂-CH₂-O)ₙ-(CH₂)ₚ-K-(CH₂)ₘ Formula (VII),

wherein:
- D: is a therapeutic agent;
- X: is an aliphatic, aromatic or heterocyclic group, preferably a carbonyl- (C=O), amine- (NH), amide- (CONH), amide- (NHCO), ester- (O-CO), carbamate-, urethane- (O-CO-NH), succinimide derivate- (S-succinimide-CH₂-CH₂-NH-CO), oxime- (C=N-O), Schiff-base- or a hydrazide- (CH=N-NH-CO) linkage; preferably a NH-CO-, a CH=N-NH-CO-, a O-CO-, or a succinimide derivate- (S-succinimidyl-CH₂-CH₂-NH-CO) linkage;
- K: is a NHCO- or a CONH- linkage;
- m: is an integer from 1 to 10, preferably 2 to 6, most preferably 2 to 4;
- n: is a integer from 0 to 400, preferably 0 to 100, more preferably 0 to 48 and most preferably 4 to 24;
- p: is an integer from 1 to 10, preferably 2 to 6, and
wherein
R₂ represents

Fᵢ-(CH₂)_{c}-K-(CH₂-CH₂-O)_{q}-(CH₂)ₘ-Y Formula (VI)

or

Fᵢ-(CH₂)_{c}-K-(CH₂-CH₂-O)q-(CH₂)_{f}-K-(CH₂-CH₂-O)ᵣ-(CH₂)ₘ-Y Formula (VIII),

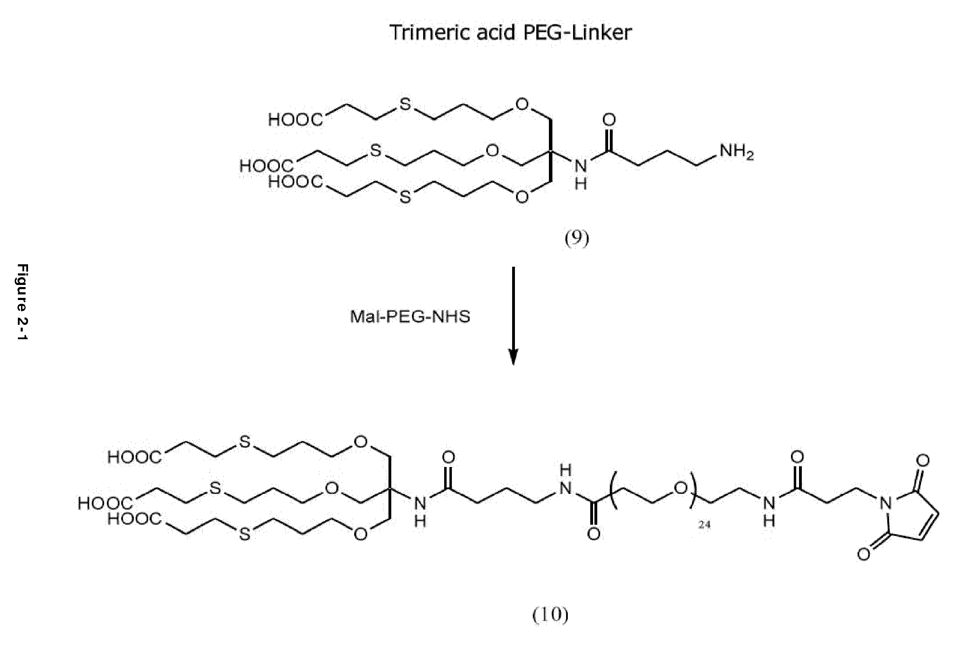

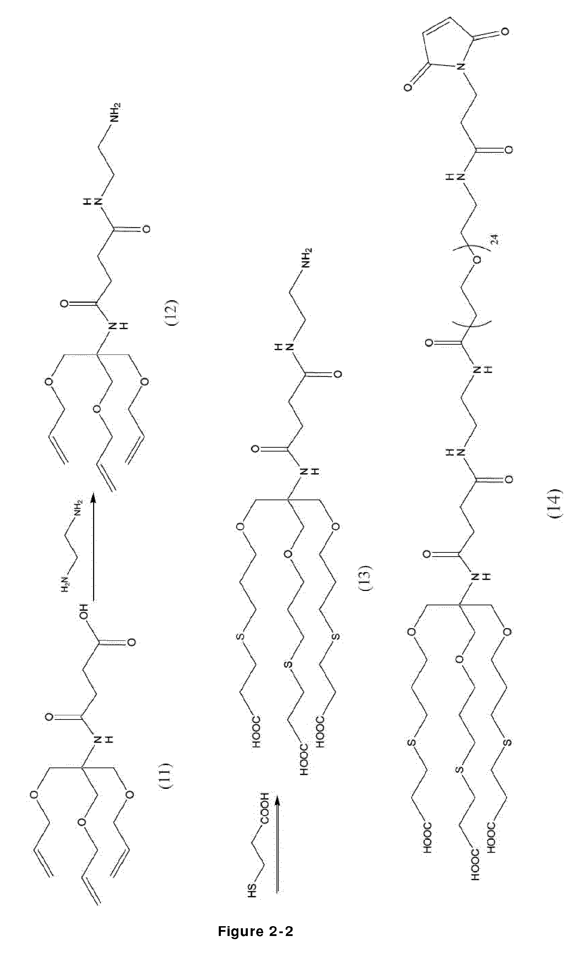

wherein
- F: is a NH- or a O- linkage;
- i: is 0 or 1;
- c: is an integer from 1 to 10;
- K: is a NHCO- or a CONH- linkage;
- q: is an integer from 0 to 400, preferably 0 to 100, more preferably 0 to 48 and most preferably 4 to 24;
- f: is an integer from 0 to 10, preferably 2 to 4; and
- r: is an integer from 0 to 400, preferably 0 to 100, more preferably 0 to 48 and most preferably 4 to 24;
- m: is an integer from 1 to 10, preferably 2 to 4; and
- Y: is an aliphatic, aromatic or heterocyclic binding group that facilitates the covalent coupling to biological targeting molecules selected from the group consisting of proteins, peptides, carbohydrates or oligonucleotides, in particular antibodies, antibody fragments, scaffold proteins, serum proteins, receptor binding proteins, nanobodies, lectins, haptenes, polymers and natural products. Therefore, in general Y fulfils the function of subsequent coupling of the conjugates according to the invention to the biological targeting molecule i.e., the compounds according to the invention preferably contain a chemically reactive functionality Y. Of course, the chemical functionality Y can be reactive per se or can be activated prior to the coupling reaction or can be activated in situ in order to facilitate the respective coupling to the biological targeting molecule. In addition, e.g. dependent on the synthesis strategy a multimeric agent according to formula (13) (see Figure 2)
can be synthesized in order to couple the therapeutic agents D and later on the respective biological targeting molecule.

In particular, Y is selected from a group of compounds having a chemically reactive functionality that reacts chemically with an amino group, a thiol group, a carboxyl group, a guanidine group, a carbonyl group, a hydroxyl group, a hydrazine group, an alkyne group, a heterocyclic group, C-nucleophile group, a C-electrophile group, a phosphate or a sulfate of the biological targeting molecule, or can form a chelate or a complex with metals, or can enter into a bond with surfaces like plastics, gold, copper, or silicon in order to be associated or physically bound via van der Waals forces or ion bindings to the biological targeting molecule.

Preferably, Y is a functional chemical group that is reactive for an amino group, a thiol group, a carboxyl group, a guanidine group, a carbonyl group or a hydroxyl group of the biological targeting molecule. Furthermore, Y can be reactive for heterocycles that contain N (e.g. Histidine residues), C-nucleophilic or C-electrophilic groups, phosphates or sulphates of the biological targeting molecule.

In detail, preferably the chemically reactive functionality Y is selected from the group consisting of a carbonic acid, an activated carbonic acid, an activated carbonic acid ester, a carbonate, an aldehyde, an epoxide, an azide, an alkine, a cyclooctine, a halogen, a vinylsulfone and a Michael acceptor system, such as e.g. (O-alkyl)2, -OSO₂CH₂CF₃ (tresyl), (O-aryl)-azides, (O-alkyl)-azides, O-alkyne -CO-Q, maleimidyl, -O-CO-nitrophenyl or trichlorophenyl, -S-S-alkyl, -S-S-aryl, - SO₂-alkeny (vinylsulfone), or -halogen (Cl, Br or I), where Q is selected independently from the group consisting of H, O-aryl, O-benzyl, O-N-succinimide, ON-sulfosuccinimide, O-N-phthalimide, O-N-glutarimide, O-N-tetrahydrophthalimide, N-norbornene-2,3-dicarboximide, hydroxybenzotriazoles and hydroxy-7-azabenzotriazoles.

More preferably Y is a aldehyde, an activated carbonic acid ester such as a N-hydroxysuccinimide ester, benzotriazole ester, pentafluorophenyl ester, maleimidyl ester, dithiopyridyl ester; a thiol (-SH), p-nitrophenyl carbonate, a (-O-CH₂-epoxy)-group, a (-O-CH₂-alkyne)-group, a -NH-CO-alkyne group, -CO-NH-alkyne group, an -azide group, a -NH-CO-CH₂-O-cyclo-di-fluorooctane group, a halogen group being preferably iodine, bromine or chlorine, a vinylsulfone (-O-S(=O)₂-CH=CH₂) group, a -NH-CO-CH₂-CH₂-halogen group, wherein preferably the halogen is iodine, bromine or chlorine, a 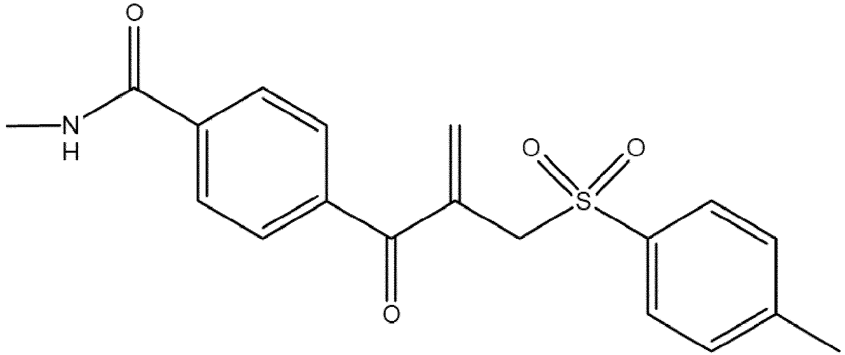 group, a 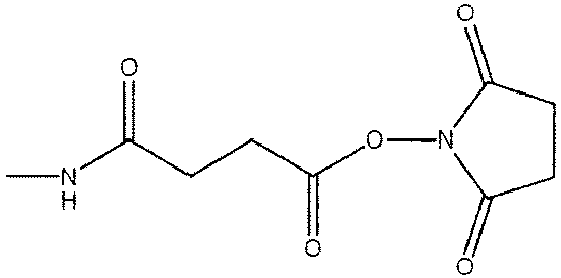 group or a 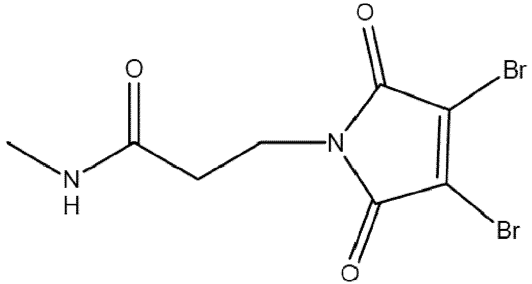 group.

Most preferably, Y is an aldehyde, an activated carbonic acid ester such as a pentafluorophenyl ester, maleimidyl ester, dithiopyridyl ester; a -NH-CO-alkyne group, an -azide group, a vinylsulfone (-O-S(=O)₂-CH=CH₂) group or a 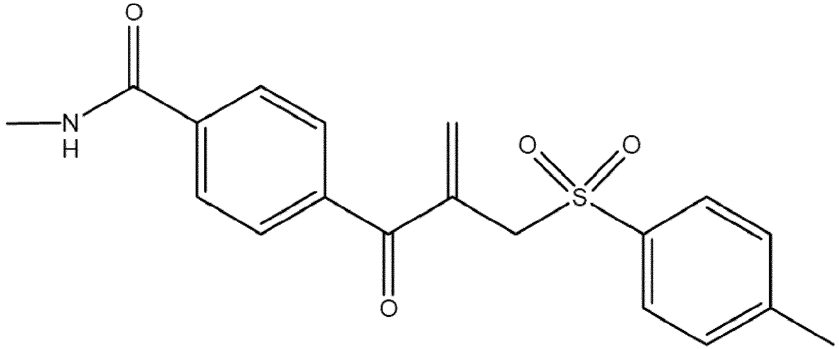 group.

The review by Zalipsky, S., which appeared in Bioconjugate Chem. 1995, 6, 150-165, provides a good overview of possible activations. This review is incorporated herein in its entirety by reference. Furthermore, Y can be a group that insert into a disulfide bridge such as Brommaleimide (Baker et al. J. Am. Chem. Soc. 2010; 132; 1960-1965) or unsaturated Arylsulfones (Brocchini et al. Nature Chemical Biology 2006; 2; 312-313). Further, common principles of activation that are known by a skilled person in the art such as e.g. described in the textbook of Miklos Bodanszky "Principles of Peptide Synthesis" (Springer Verlag, New York Berlin Heidelberg, ISBN 3-540-56431-4 and ISBN 0-387-56431-4; 2nd edition, 1993) and "The Practice of Peptide Synthesis", (Springer Verlag, New York Berlin Heidelberg, ISBN 3-540-57505-7 and ISBN 0-387-57505-7; 2nd edition, 1994) can be used.

In formula II, R₃ represents:

D-Xᵢ-(CH₂-CH₂-O)ₛ-(CH₂)_{b}-Z Formula (V)

wherein
- D: is a therapeutic agent;
- X: is an aliphatic, aromatic or heterocyclic group, preferably a carbonyl- (C=O), amine- (NH), amide- (CONH), amide- (NHCO), ester- (O-CO), carbamate-, urethane- (O-CO-NH), succinimide derivate- (S-succinimide-CH₂-CH₂-NH-CO), oxime- (C=N-O), Schiff-base- or a hydrazide- (CH=N-NH-CO) linkage; most preferably a NH-CO-, a CH=N-NH-CO-, a O-CO-, or a succinimide derivate- (S-succinimidyl-CH₂-CH₂-NH-CO) linkage;
- i: is 0 or 1;
- s: is an integer from 0 to 400, preferably 0 to 100, more preferably 0 to 48 and most preferably 4 to 24;
- b: is an integer from 0 to 10, preferably 2 to 4;
- Z: is a NH- or O- linkage, if b = 0 then Z is omitted.

The central branching unit of the conjugates according to formula (I) and (II) is based on a Tris-(hydroxymethyl)-aminomethan (TRIS) core. Attached to the branching molecule are three molecules of the therapeutic agent D comprised by the residues R₁ or R₃ and residue R₂ suitable for the coupling to a biological targeting molecule. In a preferred embodiment the conjugates according to formula (I) and (II) contain 1 to 4 polyethylene glycol (PEG) moieties, preferably 1 or 3. Water solubility of the conjugates can be adjusted by number and molecular weight of the PEG moieties. The molecular weight of the PEG moieties is defined by n, q, s and r wherein n, q, s and r are independently from each other an integer from 0 to 400, preferably 0 to 100, more preferably 0 to 48 and most preferably 4 to 24.

In substituent R₁ and R₃ of formula (I) and (II) D represents a therapeutic agent. The therapeutic agent D is an aliphatic, aromatic or heterocyclic compound that also can comprise a radionuclide. The molecular weight of the therapeutic agent D is from about 100 to about 10000 Dalton, preferably about 100 to about 5000 Dalton, more preferably about 100 to about 2000 Dalton and most preferably about 100 to about 1000 Dalton.

In a preferred embodiment the therapeutic agent D is selected from the group consisting of cytotoxic, anti-inflammatory, antibiotic and anti-analgetic agents. The therapeutic agent D can be a biotechnological, natural or synthetic compound. In preferred embodiments the therapeutic agent D is selected from the group consisting of linear or cyclic peptides, alkaloids, anthracyclines, carbohydrates, lipids and nucleic acids. In a preferred embodiment the therapeutic agent D is a cytotoxic agent.

The cytotoxic agent or cytotoxic compound is, for instance, an alkylating agent, a DNA intercalating agent, a cytotoxic antibiotic, a Vinca alkaloid, a taxane, a topoisomerase inhibitor, an antimetabolite, an amatoxin, an aromatase inhibitor or a microtubule inhibitor. In a preferred embodiment the cytotoxic agent is Dacarbazine, Temozolomide, Paclitaxel, Cabazitaxel, Docetaxel, Nocodazole, Carmustine, Vincristine, Vinblastine, Vinorelbine, Bleomycin, Mitomycin, Dactinomycin, Melphalan, Chlorambucil, Estramustine, Doxorubicin, Idarubicin, Daunorubicin, Epirubicin, Mitoxantrone, Irinotecan, Camptothecin, SN38, Etoposide, Teniposide, Methotrexate, Lometrexol, LY309887, OSI-7904L, Plevitrexed, Edatrexate, Raltitrexed, Trimetrexate, Pralatrexate, Pemetrexed, Cytarabine, Imatinib, Dasatinib, Nilotinib, SGX393, Cladribine, Gemcitabine, Epothilone, Tubulysine, Auristatin, Maytansin, Duocarmycin, PBD dimer or Erubilin, more preferably Paclitaxel, Cabataxel, Docetaxel, Vincristine, Viblastine, Mitomycin, Doxorubcin, Epirubicin, Daunorubicin, Etoposide, Teniposid, Epothilone, Tubulysine, Auristatin, Maytansin, Methotrexate, Lometrexol, LY309887, OSI-7904L, Plevitrexed, Edatrexate, Raltitrexed, Trimetrexate, or Pralatrexate and most preferably Paclitaxel, Doxorubicin, Methotrexate, Trimetrexate, Pralatrexate, Auristatin, SN38, Epothilone, Tubulysine and Maytansin.

In a preferred embodiment, the conjugate according to the invention has a structure according to formula (I) or (II), wherein R1 has a structure according to formula (III) or (VII); R2 has a structure according to formula (VI), and R3 has a structure according to formula (V), wherein
- D: is Paclitaxel, Doxorubicin, Methotrexate, SN38, Epothilone, Tubulysine or Maytansin;
- X: is a CO- or NH-linkage,
- K: is a NHCO- or CONH-linkage,
- m: is 1,2 or 3,
- n: is 1,2 or 3,
- p: is 1, 2 or 3,
- i: is 0,
- c: is 1, 2 or 3,
- q: is 0 to 30, preferably 2 to 24,
- s: is 0,1,2 or 3, preferably 0
- b: being 0 and
- Y: is an aldehyde (COH), a N-hydroxy succinimide ester (NHS-Ester), a benzotriazole ester, a pentafluorophenyl ester, a maleimidyl ester, a dithiopyridyl ester, a thiol (SH), a p-nitrophenyl carbonate, a (O-CH2-epoxy)-group, a (O-CH₂-alkyne)-group, a NH-CO-alkyne, a CO-NH-alkyne, an azide, a NH-CO-CH₂-O-cyclo-difluoro octane, a halogen, a vinylsulfone (O-S(=O)₂-CH=CH₂) group, a NH-CO-CH₂-CH₂-halogen group, preferably the halogen is iodine, bromine or chlorine, a 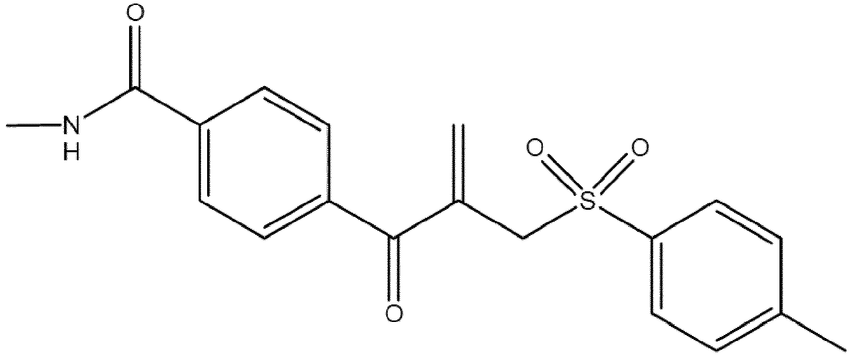
group, a 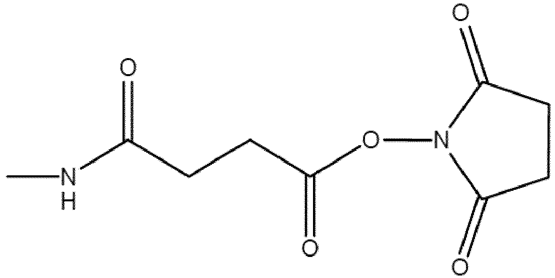 group or a 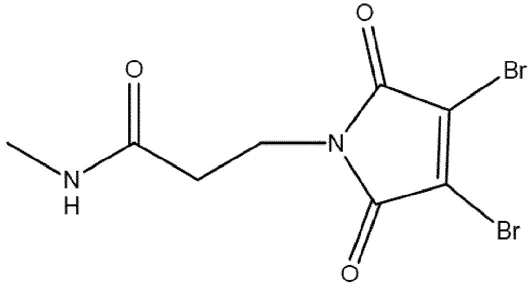 group.

In a further preferred embodiment the conjugate according to the invention has the structure of formula (I) or (II) wherein R₁ has a structure according to formula (III) or (VII); R₂ has a structure according to formula (VI), and R₃ has a structure according to formula (V), wherein D is Paclitaxel, SN-38, or Methotrexate; X represents a CO- or NH-linkage; K is a NHCO- or CONH-linkage, Y is a is an aldehyde (COH), a N-hydroxysuccinimide ester, a benzotriazole ester, a pentafluorophenyl ester, a maleimidyl ester, a dithiopyridyl ester, a thiol (SH), a p-nitrophenyl carbonate, a (O-CH2-epoxy)-group, a (O-CH₂-alkyne)-group, a NH-CO-alkyne group, CO-NH-alkyne group, an azide, a NH-CO-CH2-O-cyclo-di-fluorooctane group, a halogen group, a vinylsulfone (O-S(=O)₂-CH=CH₂) group, a NH-CO-CH₂-CH₂-halogen group, a 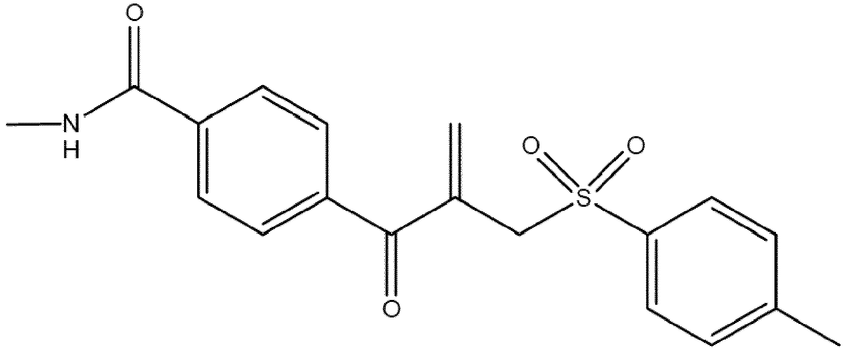 group, a 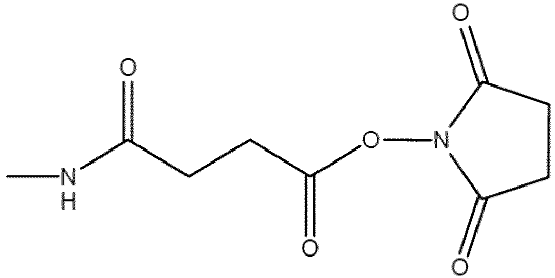 group or a 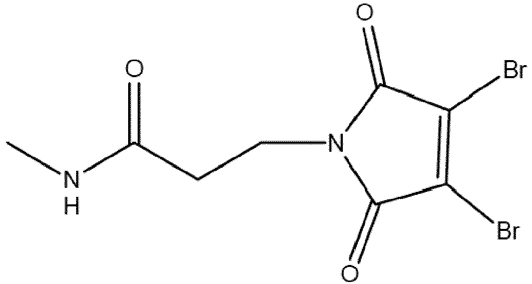 group.
and
m is 2, 3, 4, or 5;
n is 3,
p is 1,2,3, 4 or 5,
q is 1 to 24; preferably 1 or 24
i is 0 or 1;
c is 2, 3, 4 or 5; and
b is 3,
f is 2, and
Z is a NH-group.

In a most preferred embodiment the conjugates according to the invention have the chemical structure of formulae (4) and (17) (see Figure 3), formulae (5) and (6) (see Figure 4), formulae (7) and (8) (see Figure 5) and the last formula in Figure 6.

In another embodiment the invention relates to a Methotrexate-linker-conjugate-human serum albumin compound, wherein the chemical group Y of the Methotrexate-linker-conjugate according to the invention having formula (I) or (II), wherein D of R1 or R3 is Methotrexate, was chemically reacted with at least a thiol group of a Cysteine residue of human serum albumin or the amino group of a Lysine residue of human serum albumin, preferably a Cysteine residue and most preferably the Cystein-34 of human serum albumin. This chemical linkage makes sure that the biological structure of human serum albumin will be maintained after the chemical reaction within the Methotrexate-linker-conjugate-human serum albumin compound. In general, Y of R₂ can be an aldehyde (COH), a N-hydroxy succinimide ester (NHS-Ester), a benzotriazole ester, a pentafluorophenyl ester, a maleimidyl ester, a dithiopyridyl ester, a thiol (SH), a p-nitrophenyl carbonate, a (O-CH2-epoxy)-group, a (O-CH₂-alkyne)-group, a NH-CO-alkyne, a CO-NH-alkyne, an azide, a NH-CO-CH₂-O-cyclo-di-fluoro octane, a halogen, a vinylsulfone (O-S(=O)₂-CH=CH₂) group, a NH-CO-CH₂-CH₂-halogen group, preferably the halogen is iodine, bromine or chlorine, a 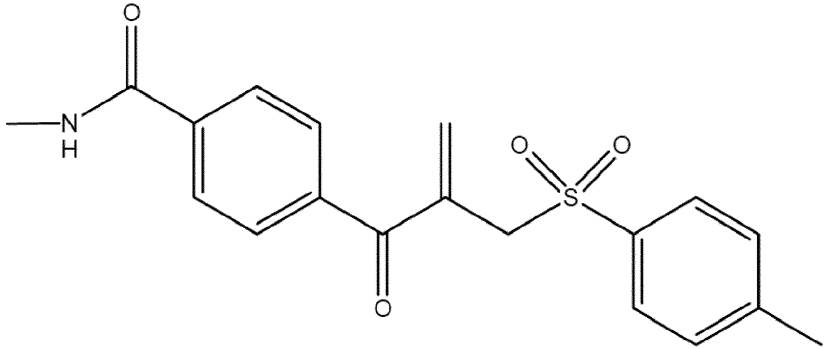 group, a 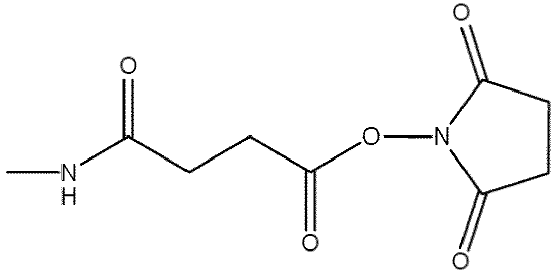 group or a 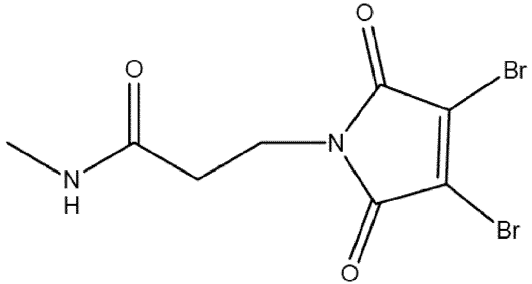 group.

Preferably, in formula (I) or (II), R₁ has a structure according to formula (III) or (VII); R₂ has a structure according to formula (VI), and R₃ has a structure according to formula (V), wherein D is Methotrexate; X represents a CO- or NH-linkage; K is a NHCO- or CONH-linkage, Y is a is an aldehyde (COH), a N-hydroxysuccinimide ester, a benzotriazole ester, a pentafluorophenyl ester, a maleimidyl ester, a dithiopyridyl ester, a thiol (SH), a p-nitrophenyl carbonate, a (O-CH₂-epoxy)-group, a (O-CH₂-alkyne)-group, a NH-CO-alkyne group, CO-NH-alkyne group, an azide, a NH-CO-CH2-O-cyclo-di-fluorooctane group, a halogen group, a vinylsulfone (O-S(=O)₂-CH=CH₂) group, a NH-CO-CH₂-CH₂-halogen group, a 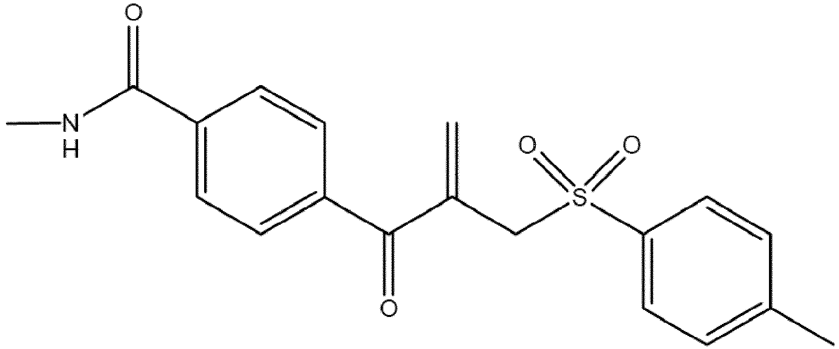 group, a 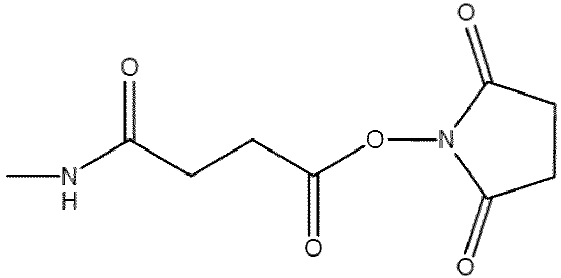 group or a 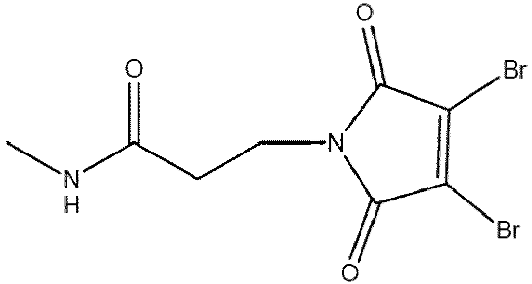 group.

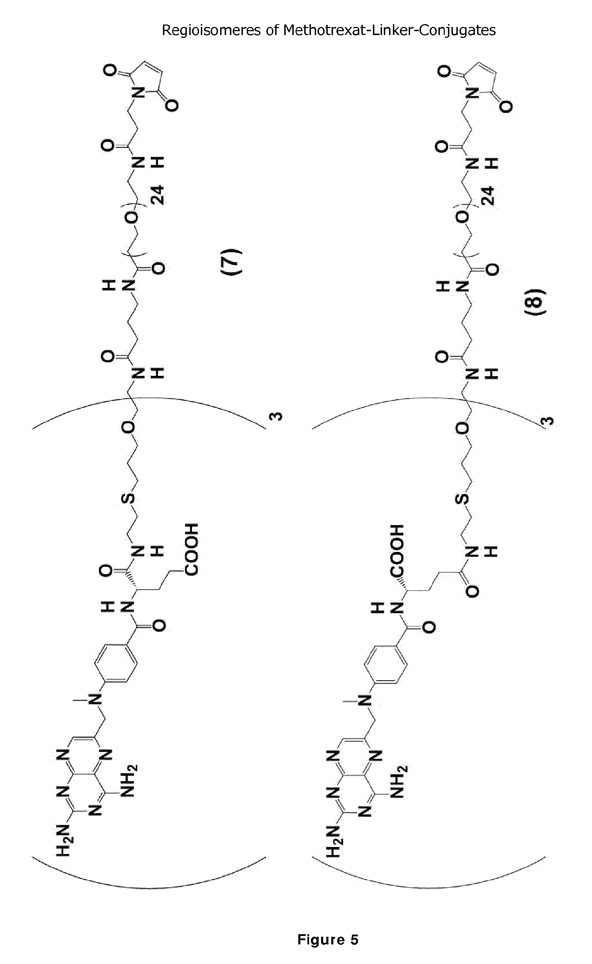

In a preferred embodiment the Methotrexate-linker-conjugate is a Methotrexate-linker-conjugate according to formula (7) and/or (8) as shown in Figure 5. Figure 5 shows the regioisomeres of a Methotrexate-Linker-Conjugate. Both regioisomers can be used alone or as a mixture in all ratios. Y of residue R2 has a maleimide-functional part, i.e. -HNCO-CH₂-CH₂-maleimidyl that reacts in a Michael addition with the thiol function of a Cysteine residue of human serum albumin or the amino group of a Lysine residue of human serum albumin, preferably a Cysteine residue and most preferably the amino acid 34 that is a 34-cysteine to the respective Methotrexate-linker-conjugate-human serum albumin compound.

The invention further relates to a pharmaceutical composition comprising at least a conjugate according to formula (I) or (II) or a pharmaceutically acceptable salt or solvate thereof and optionally pharmaceutically acceptable excipients, carriers or diluents.

In addition, the invention relates to a pharmaceutical kit comprising (i) at least a conjugate according to formula (I) or (II) or a pharmaceutical composition according to the invention and (ii) an anti-inflammatory, anti-viral or antiproliferative drug.

The conjugate, pharmaceutical composition or pharmaceutical kit according to the invention can be used as a medicament or a tool in biomedical research.

Further, the conjugate, pharmaceutical composition or pharmaceutical kit according to the invention can be used for the treatment of a disease or disorder that can be at least in part alleviated by therapy. In this respect, the disease or disorder is selected from the group consisting of inflammatory, auto-inflammatory or infectious disease or disorders, rheumatoid arthritis or cancer.

### BRIEF DESCRIPTION OF THE DRAWING

The figures presented herewith and the attendant examples are not to be construed as limiting the invention in any way, but rather as serving only for the purpose of exemplification.

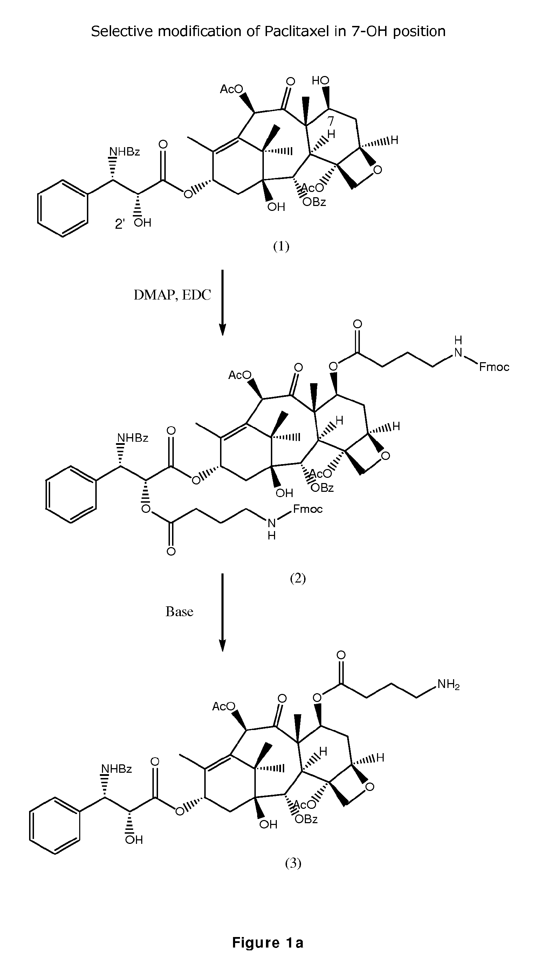

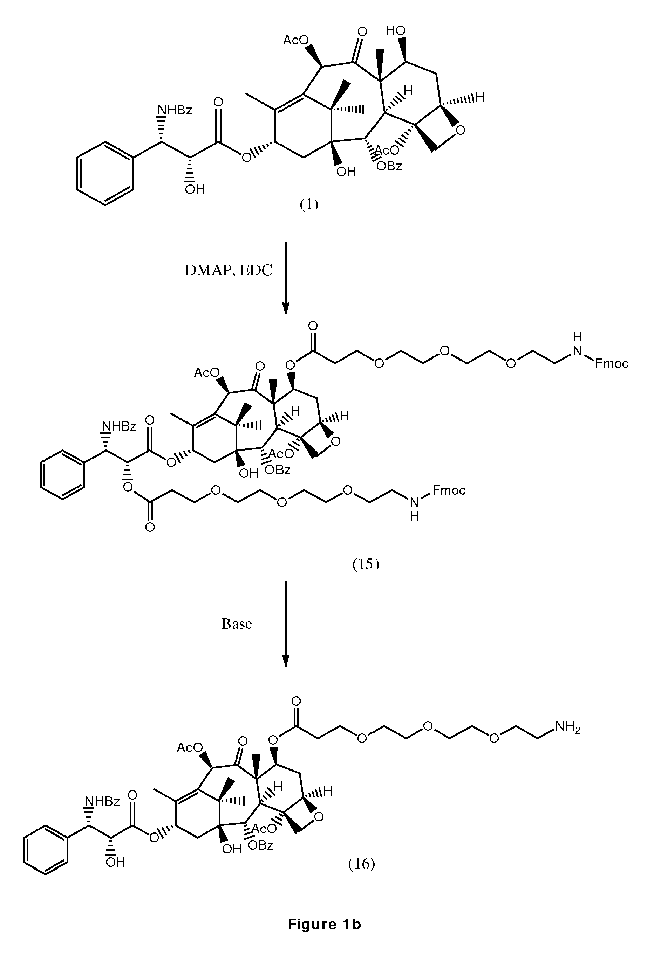

Figures 1a and 1b show two examples of the synthesis of a selective modification of Paclitaxel in 7-OH position.
Figure 2 shows the synthesis of a trimeric acid PEG-Linker.

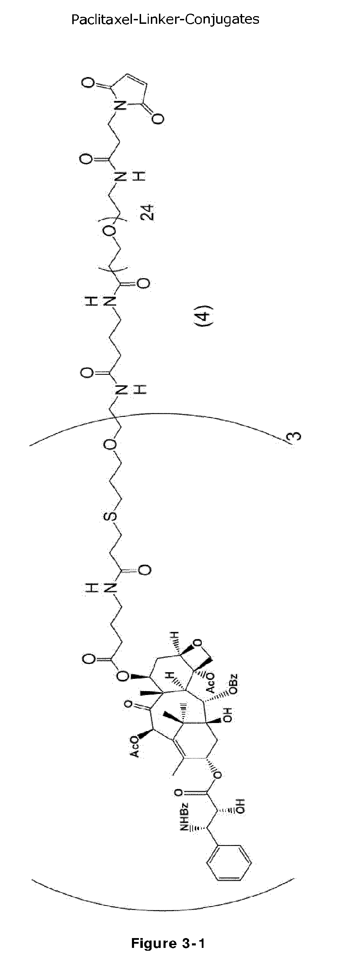

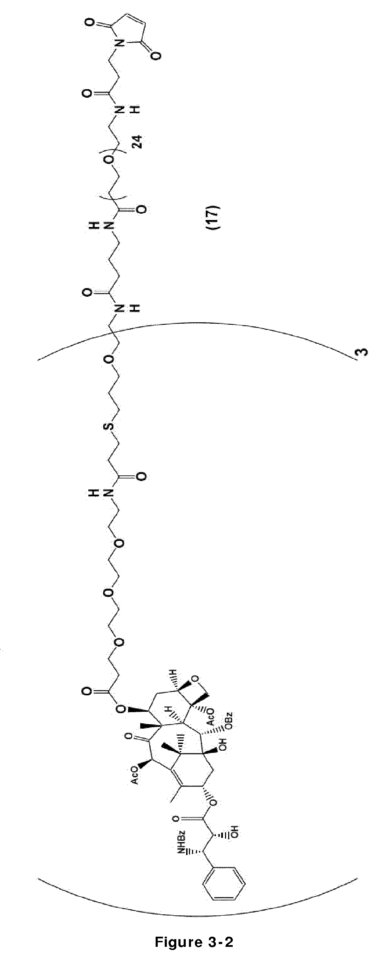

Figure 3 shows two different Paclitaxel-Linker-Conjugates.

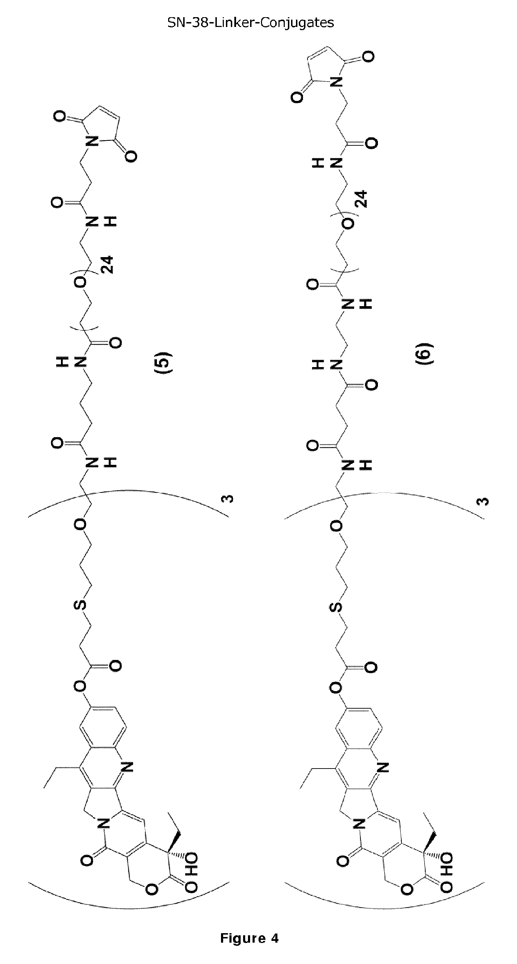

Figure 4 shows two different SN-38-Linker-Conjugates.
Figure 5 shows the regioisomeres of a specific Methotrexate-Linker-Conjugate.

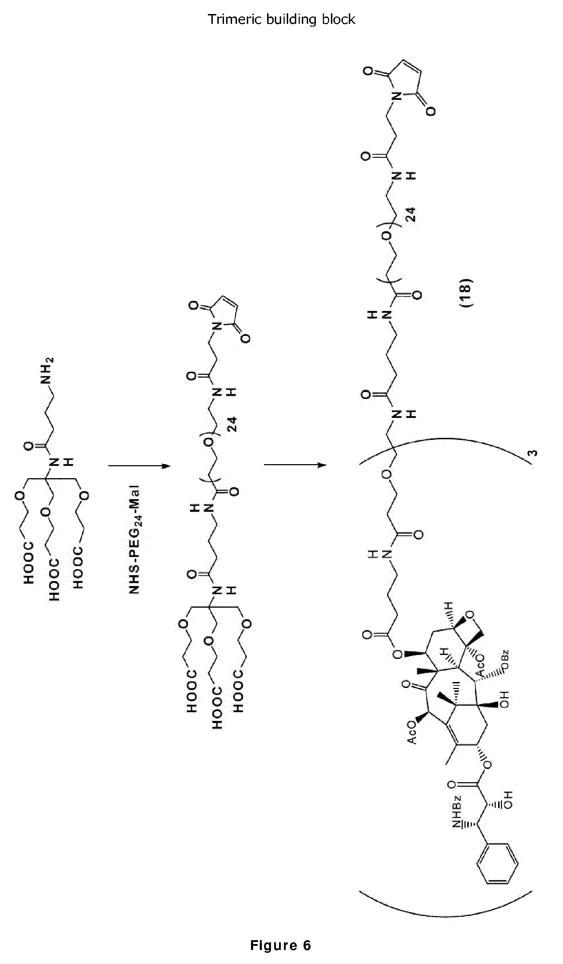

Figure 6 shows the synthesis of a trimeric Paditaxel-Linker-Conjugate.

### DESCRIPTION OF SYNTHESIS

The present invention further relates to a short, efficient procedure for selective modification of Paclitaxel at the 7-OH position. This process facilitates the preparation of water soluble, trimeric Paclitaxel-Linker-Conjugates according to formula (I) or (II) of the present invention. As already described, these conjugates can be used to produce a pharmaceutical composition or a pharmaceutical kit. Based on the higher reactivity Paclitaxel is usually esterified at the 2'-OH position as disclosed also in US 6,649,778 or the OH-functional group has to be modified by a protecting group, which tends to result in extra process steps. Furthermore, the 7-OH esters are significantly more stable in aqueous solution, thus they are highly suitable for application in antibody-drug-conjugates.

Conjugates according to formula (I) or (II) of the present invention can be coupled to biological targeting molecules selected from the group of proteins, peptides, carbohydrates or oligo-nucleotides, in particular antibodies, antibody fragments, scaffold proteins, serum proteins, receptor binding proteins, nanobodies, lectins, haptenes, polymers and natural products within one reaction step.

The coupling is performed in an environment compatible with the biological targeting molecule. Conjugates according to the invention are very good soluble in aqueous solutions and do not require organic solvents or detergents. This facilitates mild reaction conditions and in this way prevents aggregation or inactivation of the biological targeting molecule. In preferred embodiments, conjugates according to the present invention are coupled to the biological targeting molecule in a one reaction step. The reaction takes place in aqueous buffers with pH values between about 2 and about 12, more preferably between about 4 and about 10 and most preferably between about 5 and about 9.

Thus, conjugates of the present invention can be used to produce therapeutic drugs with beneficial pharmacological properties that are very specific and show a high therapeutic effect for a target tissue, respectively. In preferred applications such therapeutic drugs are used for the treatment of inflammatory or auto-inflammatory diseases, cancer, rheumatoid arthritis or infectious diseases.

The present invention further relates to a method for the preparation of a Paclitaxel- or Paclitaxel derivatives linker-conjugate according to formula (I) or (II) of claim 1, wherein R1 is D-X-(CH₂-CH₂-O)ₙ-(CH₂)ₚ-K-(CH₂)ₘ (Formula VII),
wherein
- D: is the therapeutic agent being Paclitaxel linked via the 7'OH group of Paclitaxel to X;
- X: a carbonyl- (C=O) group;
- n: is 0;
- p: is from 1 to 10, preferably from 2 to 6, most preferably 2, 3 or 4;
- K: is a NHCO- linkage;
- m: is from 1 to 10, preferably from 2 to 6, most preferably 2, 3 or 4; and
R2 represents

Fᵢ-(CH₂)_{c}-K-(CH₂-CH₂-O)_{q}-(CH₂)ₘ-Y Formula (VI)

or

Fᵢ-(CH₂)_{c}-K-(CH₂-CH₂-O)_{q}-(CH₂)_{f}-K-(CH₂-CH₂-O)ᵣ-(CH₂)ₘ-Y Formula (VIII)

wherein
- i: is 0;
- c: is 3;
- q: is 1 or 24;
- f: is 2;
- K: is a NHCO-linkage;
- r: is 0;
- m: is 2; and
- Y: is is a aldehyde (COH), a N-hydroxysuccinimide ester, a benzotriazole ester, a pentafluorophenyl ester, a maleimidyl ester, a dithiopyridyl ester, a thiol (SH), a p-nitrophenyl carbonate, a (O-CH2-epoxy)-group, a (O-CH₂-alkyne)-group, a NH-CO-alkyne group, a CO-NH-alkyne group, an azide, a NH-CO-CH₂-O-cyclo-di-fluorooctane group, a halogen group, a vinylsulfone (O-S(=O)₂-CH=CH₂) group, a NH-CO-CH₂-CH₂-halogen group, a 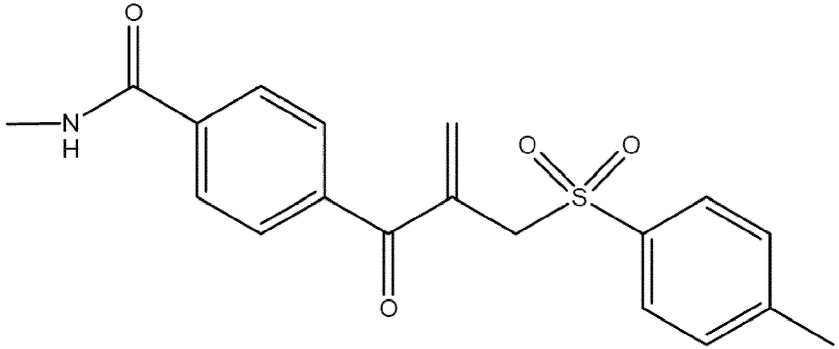
group, a 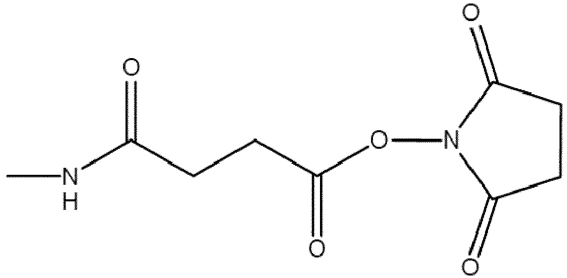 group or a 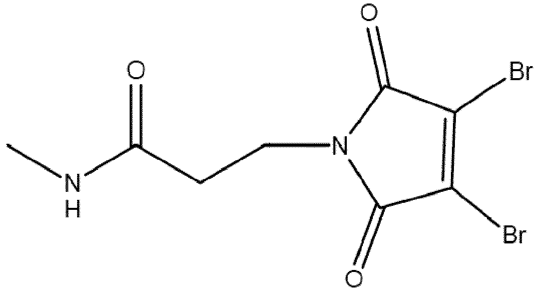 group comprising the steps of
(a) reacting a Paclitaxel or a Paclitaxel derivatives with the respective equivalents of a Fmoc amino protected linear amino alkanoic acid to obtain an 2'OH- and 7'OH-Fmoc di-protected Paclitaxel derivative, wherein the linear amino alkanoic acid is selected from the group consisting of glycine, aminopropionic acid (beta-alanine), aminobutyric acid, aminovaleric acid, aminocapronic acid, aminoenanthic acid, aminocyprylic acid, aminopelargonic acid, aminocapric acid and aminoundecylic acid, preferably glycine, aminopropionic acid (beta-alanine) and aminobutyric acid, most preferably aminobutyric acid;
(b) cleaving of the 2'OH-Fmoc amino protected linear amino alkanoic acid and deprotecting of the 7'OH Fmoc protecting group by treatment with a base, preferably a non-nucleophilic amino base, like e.g. Triethylamine (TEA), 1,8-Diazabicyclo[5.4.0]undec-7-ene (DBU), or N,N-Diisopropylethylamin (Hünig's base); to obtain the linear amino alkanoic acid derivative of Paclitaxel or the Paxlitaxel derivates linked via the 7'OH of Paclitaxel or the Paclitaxel derivative; preferably e.g. compound (16);
(c) reacting the compound of step (b) with the reaction product of a trimeric acid linker having the structural elements of formula (I) or (II) as defined above, like e.g. compound (14) (see Figure 2) in order to obtain said Paclitaxel- or Paclitaxel derivatives linker-conjugate, which is preferably e.g. compounds (4) or (17) (see Figure 3) or compound (18) (see Figure 6).

### EXAMPLES

### Preparation of Paclitaxel 7-OH derivatives

**Amino acid derivative of Paclitaxel** (**3**): To a mixture of Paclitaxel (**1**) (1.01 g, 1.18 mmol), Fmoc-gamma aminobutyric acid (978 mg, 3.01 mmol) and Dimethylaminopyridine (724 mg, 5.93 mmol) dissolved in Dichloromethane (100 ml) 1-Ethly-3-(3-dimethylaminopropyl)carbodiimide (1.13 g, 5.91 mmol) was added at 20-25°C. The resulting, colorless reaction mixture was stirred for 24 hrs at 20-25°C. Afterwards the reaction mixture was washed with aqueous sodium bicarbonate (60 ml, 100 mM). Organic solvent was evaporated to give crude (2) as an off-white solid (purity > 95 area% HPLC-ELSD). The crude product (2) was dissolved in Dimethylformamide (70 ml), followed by adding Dimethylaminopyridine (2.93 g, 23.98 mmol) and 1,4-Dithioerythritol (1.11 g, 7.18 mmol) at 20-25°C. The reaction mixture was stirred for 20 hrs at 20-25°C. Subsequently, the reaction mixture was diluted by Ethyl acetate (100 ml), washed with an aqueous acetic acid solution (200 ml, 1 %) and the separated aqueous phase was extracted three times with Dichloromethane (50 ml). The combined organic layers were concentrated in vacuo. The residual crude product was purified by preparative RP-MPLC using water and Acetonitrile (MeCN) as eluents (gradient: 10 → 60 % MeCN + 0.05 % Trifluoroacetic acid). (3) was obtained (804 mg, 64 % yield) as a white amorphous solid. Structure determination was performed by two dimensional NMR spectroscopy (HSQC and HMBC).

**Trimeric acid PEG₂₄-Maleimide Linker** (**10**): The preparation of building block (9) has been already described in US2010/160409. To a solution of **(9)** (2.88 g, 3.86 mmol) in Dichloromethane (30 ml) Trifluoroacetic acid (5.95 ml, 77.21 mmol) was added at 20-25°C. This mixture was stirred for 1 hr at 20-25°C. In the following solvent was evaporated to obtain the de-protected primary amine as highly viscose oil. Without further purification a portion of the crude oil (1.53 g, 1.43 mmol) was dissolved in Dichloromethane (50 ml) followed by the addition of NHS-PEG₂₄-Mal (2.00 g, 1.43 mmol) and Triethylamine (997 µl, 7.17 mmol) at 20-25°C. Subsequently, the reaction mixture was stirred for 12 hrs at 20-25°C and afterwards the solvent was removed in vacuo. Chromatographic purification on silica (Dichloromethane + 5 % Methanol → 10 % Methanol) gave (**10**) as a colorless, vicose oil (2.49 g, 89% yield). MALDI-MS: m/z 1925.9 [M+H]⁺; molecular formula: C₈₄H₁₅₄N₄O₃₈S₃. The structure was further confirmed by NMR spectroscopy.

**Trimeric acid PEG₂₄-Maleimide Linker** (**14**): The preparation of building block (**11**) has been already described in US2010160409. To a solution of (**11**) (4.30 g, 12.60 mmol) in Dichloromethane (50 ml) Dicyclohexylcarbodiimide (3.13 g, 15.17 mmol) and N-Hydroxysuccinimide (2.61 g, 22.68 mmol) was added at 20-25°C. The reaction mixture was stirred for 12 hrs at 20-25°C. Afterwards the precipitated Dicyclohexyl urea was removed by filtration. To the stirred, clear filtrate Ethylenediamine (42 ml, 630.00 mmol) was added at 20-25°C and the mixture was stirred for further 3 hrs. In the following the reaction mixture was diluted with Dichloromethane (100 ml) and the organic layer was washed with water (100 ml). Subsequently the organic layer was concentrated in vacuo and chromatographic purification on silica (Dichloromethane + 5 % Methanol + 0.1 % NH₄OH-Lsg → Dichloromethane + 30 % Meethanol + 0.1 % NH₄OH-Lsg) gave (**12**) as a pale yellow oil (2.64 g, 55% yield). Structure determination was performed by NMR spectroscopy.

To a solution of (**12**) (1.70 g, 4.43 mmol) in Ethanol (40 ml), which was degased by ultrasound, the radical initiator Azobisisobutyronitrile (182 mg, 1.11 mmol) and 3-Mercaptopropionic acid (3.1 ml, 35.46 mmol) was added at 20-25°C. This mixture was heated to 80°C under a Nitrogen atmosphere and stirred for 2 hrs. Subsequently, the solvent was removed by rotary evaporation and chromatographic purification on silica (Dichloromethane + 10 % Methanol + 0.1 % Acetic acid) gave (**13**) (1.86 g, 60% yield) as a colorless oil. MALDI-MS: m/z 702.5 [M+Na]⁺; molecular formula: C₂₈H₅₁N₃O₁₁S₃. The structure was further confirmed by ¹H-NMR and ¹³C-NMR spectroscopy.

To a solution of (**13**) (149 mg, 212 µmol) in Dichloromethane (2 ml) a mixture of NHS-PEG₂₄-Mal (200 mg, 143 µmol) and Triethylamine (45 µl, 320 µmol) dissolved in Dimethylacetamide (2 ml) was added at 20-25°C. The reaction mixture was stirred for 5 hrs at 20-25°C. After removal of the solvent in vacuo, the oily residue was purified on silica (Dichloromethane + 10 % Methanol + 0.1 % Acetic acid → Dichloromethane + 50 % Methanol + 0.1 % Acetic acid) to obtain **(14)** as (86 mg, 30% yield) as a colorless, viscose oil. MALDI-MS: m/z 2004.4 [M+Na]⁺; molecular formula: C₈₆H₁₅₇N₅O₃₉S₃.

**Trimeric Paclitaxel-Linker-Conjugate** (4): To a solution of **(10)** (400 mg, 208 µmol) in Dichloromethane (25 ml) N-Hydroxysuccinimide (100 mg, 869 µmol) and 1-Ethly-3-(3-dimethylaminopropyl)carbodiimide (160 mg, 835 µmol) were added at 20-25°C and the resulting reaction mixture was stirred for 2 hrs. After NHS-ester formation (3) (689 mg, 655 µmol) and Triethylamine (100 µl, 722 µmol) were added and the reaction mixture was stirred for further 18 hrs at 20-25°C. In order to remove water soluble side products, the reaction mixture was washed with a citric acid solution (100 ml, 50 mM). The separated organic layer was concentrated in vacuo. Chromatographic purification of the residue by preparative RP-MPLC (50 → 100 % MeCN + 0.05 % Acetic acid) gave **(4)** (209 mg, 21% yield) as a colorless, amorphous solid. MALDI-MS: m/z 4694.0 [M+Li]⁺; molecular formula: C₂₃₇H₃₂₂N₁₀O₈₀S₃.

**Trimeric SN38-Linker-Conjugate (5):** To a mixture of **(10)** (690 mg, 359 µmol) and SN-38 (505 mg, 1.29 mmol) dissolved in Dimethylacetamide (70 ml) Diisopropylethylamine (488 µl, 2.87 mmol) and (1-[Bis(dimethylamino)methylene]-1H-1,2,3-triazolo[4,5-b]pyridinium 3-oxid hexafluorophosphate (HATU, 545 mg, 1.43 mmol) were added at 20-25°C. This reaction mixture was stirred for 24 hrs at 20-25°C. After concentration in vacuo and purification by preparative RP-MPLC (20 → 90 % MeCN) **(5)** (435 mg, 40% yield) was obtained as yellow amorphous solid. MALDI-MS: m/z 3048.8 [M+H]⁺; molecular formula: C₁₅₀H₂₀₈N₁₀O₅₀S₃.

**Trimeric SN38-Linker-Conjugate (6):** To a solution of **(14)** (39 mg, 19 µmol) and Diisopropylethylamine (25 µl, 150 µmol) in Dichloromethane (3.0 ml) a mixture of SN-38 (29 mg, 75 µmol) and HATU (29 mg, 77 µmol) dissolved in Dimethylacetamide (3.0 ml) was added at 20-25°C. The obtained reaction mixture was stirred for 24 hrs at 20-25°C. Subsequently the solvent was removed in vacuo. Chromatographic purification of the residue on silica (Dichloromethane + 1 % Methanol → 30 % Methanol) gave **(6)** (17 mg, 28% yield) as a yellow, amorphous solid. MALDI-MS: m/z 3128.2 [M+Na]⁺; molecular formula: C₁₅₂H₂₁₁N₁₁O₅₁S₃.

## Claims

1. A conjugate of formula I or II, 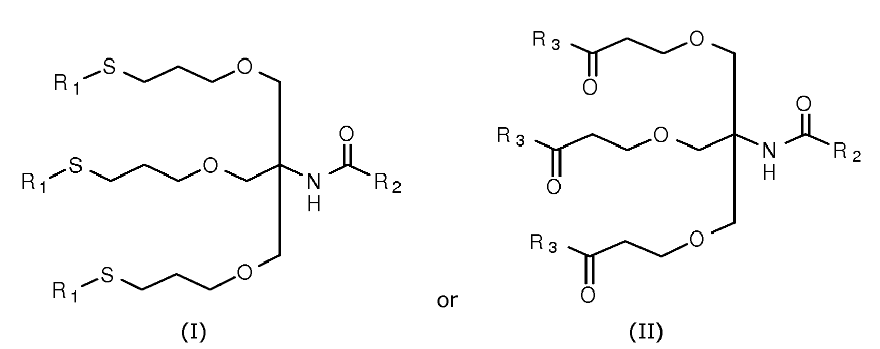 wherein
R₁ represents
D-X-(CH₂)ₘ Formula (III)
or
D-X-(CH₂-CH₂-O)ₙ-(CH₂)ₘ Formula (IV)
or
D-X-(CH₂-CH₂-O)ₙ-(CH₂)ₚ-K-(CH₂)ₘ Formula (VII),
wherein:
D is a therapeutic agent;
X is a aliphatic, aromatic or heterocyclic group, preferably a carbonyl- (C=O), amine- (NH), amide- (CONH), amide-(NHCO), ester- (O-CO), carbamate-, urethane- (O-CO-NH), succinimide derivate- (S-succinimide-CH₂-CH₂-NH-CO)-, oxime- (C=N-O), Schiff-base- or a hydrazide- (CH=N-NH-CO) linkage;
K is a NHCO- or a CONH- linkage;
m is an integer from 1 to 10;
n is a integer from 0 to 400;
p is an integer from 1 to 10; and wherein
R₂ represents
Fᵢ-(CH₂)_{c}-K-(CH₂-CH₂-O)_{q}-(CH₂)ₘ-Y Formula (VI)
or
Fᵢ-(CH₂)_{c}-K-(CH₂-CH₂-O)_{q}-(CH₂)_{f}-K-(CH₂-CH₂-O)ᵣ-(CH₂)ₘ-Y Formula (VIII),
wherein
F is a NH- or a O- linkage;
i is 0 or 1;
c is an integer from 1 to 10;
K is a NHCO- or a CONH- linkage;
q is an integer from 0 to 400;
m is an integer from 1 to 10;
f is an integer from 0 to 10;
r is an integer from 0 to 400;
Y is an aliphatic, aromatic or heterocyclic binding group that facilitates the covalent coupling to biological targeting molecules selected from the group consisting of proteins, peptides, carbohydrates or oligonucleotides; and
wherein
R₃ represents:
D-Xᵢ-(CH₂-CH₂-O)ₛ-(CH₂)_{b}-Z Formula (V)
wherein
D is a therapeutic agent;
X is an aliphatic, aromatic or heterocyclic group, preferably a carbonyl- (C=O), amine- (NH), amide- (CONH), amide-(NHCO), ester- (O-CO), carbamate-, urethane- (O-CO-NH), succinimide derivate- (S-succinimide-CH₂-CH₂-NH-CO)-, oxime- (C=N-O), Schiff-base- or a hydrazide- (CH=N-NH-CO) linkage; and
i is 0 or 1;
s is an integer from 0 to 400;
b is an integer from 0 to 10;
Z is a NH- or O- linkage, if b = 0 then Z is omitted.

2. The conjugate according to claim 1, wherein the therapeutic agent D is selected from the group consisting of cytotoxic, anti-inflammatory, antibiotic and anti-analgesic agents and preferably is a cytotoxic agent.

3. The conjugate according to claim 1 or 2, wherein the cytotoxic agent is selected from the group consisting of Dacarbazine, Temozolomide, Paclitaxel, Cabazitaxel, Docetaxel, Nocodazole, Carmustine, Vincristine, Vinblastine, Vinorelbine, Bleomycin, Mitomycin, Dactinomycin, Melphalan, Chlorambucil, Estramustine, Doxorubicin, Idarubicin, Daunorubicin, Epirubicin, Mitoxantrone, Irinotecan, Camptothecin, SN38, Etoposide, Teniposide, Methotrexate, Lometrexol, LY309887, OSI-7904L, Plevitrexed, Edatrexate, Raltitrexed, Trimetrexate, Pralatrexate, Pemetrexed, Cytarabine, Imatinib, Dasatinib, Nilotinib, SGX393, Cladribine, Gemcitabine, Epothilone, Tubulysine, Auristatin, Maytansin, Duocarmycin, PBD dimer and Erubilin.

4. The conjugate according to any of the preceding claims, wherein R₁ has a structure according to formula (III) or (VII); R₂ has a structure according to formula (VI), and R₃ has a structure according to formula (V), wherein
D is Paclitaxel, Doxorubicin, Methotrexate, SN38, Epothilone, Tubulysine or Maytansin;
X is a CO- or NH-linkage,
K is a NHCO- or CONH-linkage,
m is 1,2 or 3,
n is 1,2 or 3,
p is 1, 2 or 3,
i is 0,
c is 1, 2 or 3,
q is 0 to 30, preferably 2 to 24,
s is 0,1,2 or 3,
b being 0 or 3 and
Y is an aldehyde (COH), a N-hydroxysuccinimide ester, a benzotriazole ester, a pentafluorophenyl ester, a maleimidyl ester, a dithiopyridyl ester, a thiol (SH), a p-nitrophenyl carbonate, a (O-CH₂-epoxy)-group, a (O-CH₂-alkyne)-group, an amide- (NH-CO), an alkyne, a CO-NH-alkyne, an azide, NH-CO-CH₂-O-cyclo-di-fluorooctane, a halogen group, a vinylsulfone (O-S(=O)₂-CH=CH₂) group, a NH-CO-CH₂-CH₂-halogen group, a NH-CO-C₆H₄-CO-[C=CH₂]-CH₂-S(=O)₂-C₆H₅-CH₃ group, or a 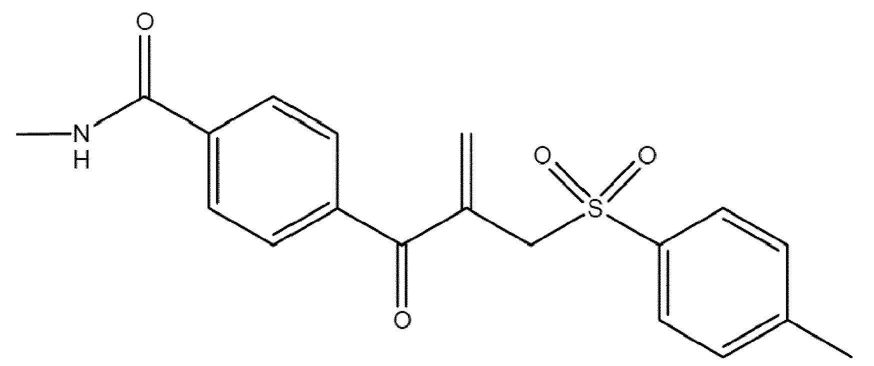
group, a 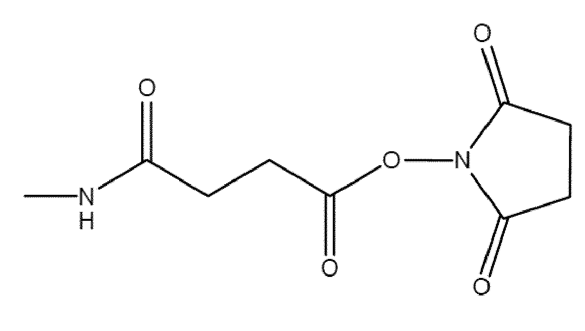 group or a 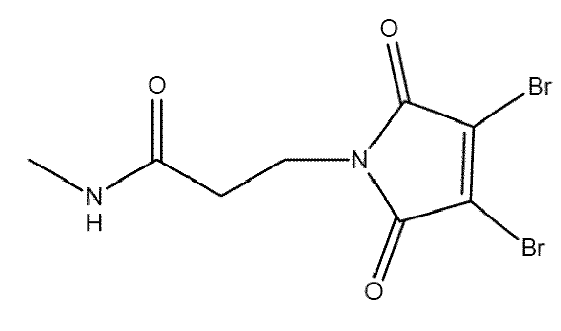 group.

5. The conjugate according to any of the preceding claims, wherein D is Paclitaxel, SN-38, or Methotrexate; X represents a CO- or NH-linkage;
K is a NHCO- or CONH-linkage, Y is a is an aldehyde (COH), a N-hydroxysuccinimide ester, a benzotriazole ester, a pentafluorophenyl ester, a maleimidyl ester, a dithiopyridyl ester, a thiol (SH), a p-nitrophenyl carbonate, a (O-CH₂-epoxy)-group, a (O-CH₂-alkyne)-group, a NH-CO-alkyne, a CO-NH-alkyne, an azide group, NH-CO-CH₂-O-cyclo-di-fluorooctane group, a halogen group, a vinylsulfone (O-S(=O)₂-CH=CH₂) group, a NH-CO-CH₂-CH₂-halogen group,
a NH-CO-C₆H₄-CO-[C=CH₂]-CH₂-S(=O)₂-C₆H₅-CH₃ group, or a 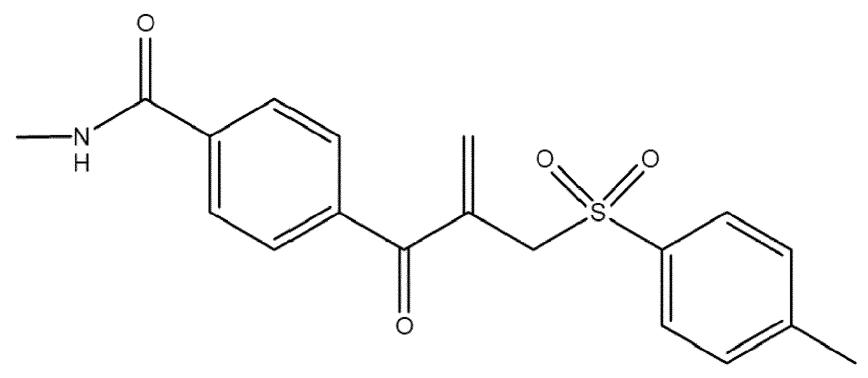 group, a 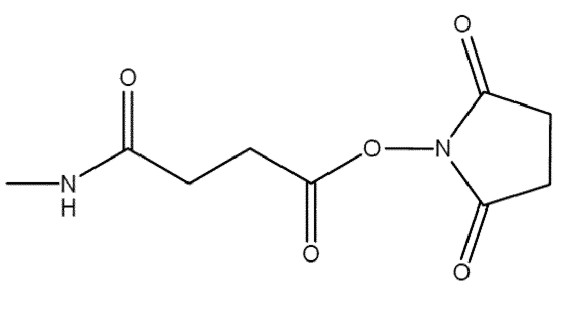 group or a 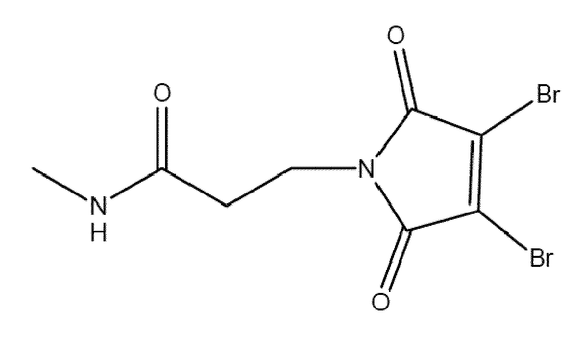 group,
and
m is 2, 3, 4, or 5;
n is 3,
p is 1,2,3, 4 or 5,
q is 1 to 24;
i is 0 or 1;
c is 2, 3, 4 or 5; and
b is 3,
f is 2, and
Z is a NH-group.

6. A method for the preparation of a Paclitaxel- or Paclitaxel derivatives linker-conjugate according to formula (I) or (II) of claim 1, wherein
R1 is D-X-(CH₂-CH₂-O)ₙ-(CH₂)ₚ-K-(CH₂)ₘ (Formula VII),
wherein
D is the therapeutic agent being Paclitaxel linked via the 7'OH group of Paclitaxel to X;
X is a carbonyl- (C=O) group;
n is 0;
p is from 1 to 10;
K is a NHCO- linkage;
m is from 1 to 10; and
R2 represents
Fᵢ-(CH₂)_{c}-K-(CH₂-CH₂-O)_{q}-(CH₂)ₘ-Y Formula (VI)
or
Fᵢ-(CH₂)_{c}-K-(CH₂-CH₂-O)_{q}-(CH₂)_{f}-K-(CH₂-CH₂-O)ᵣ-(CH₂)ₘ-Y Formula (VIII)
wherein
i is 0;
c is 3;
q is 1 or 24;
f is 2;
K is a NHCO-linkage;
r is 0;
m is 2; and
Y is is an aldehyde (COH), a N-hydroxysuccinimide ester, a benzotriazole ester, a pentafluorophenyl ester, a maleimidyl ester, a dithiopyridyl ester, a thiol (SH),a p-nitrophenyl carbonate, a (O-CH₂-epoxy)-group, a (O-CH₂-alkyne)-group, an amide- (NH-CO) group, an alkyne group, a CO-NH-alkyne group, an azide group, NH-CO-CH₂-O-cyclo-di-fluorooctane group, a halogen group, a vinylsulfone (O-S(=O)₂-CH=CH₂) group, a NH-CO-CH₂-CH₂-halogen group, a NH-CO-C₆H₄-CO-[C=CH₂]-CH₂-S(=O)₂-C₆H₅-CH₃ group, or a 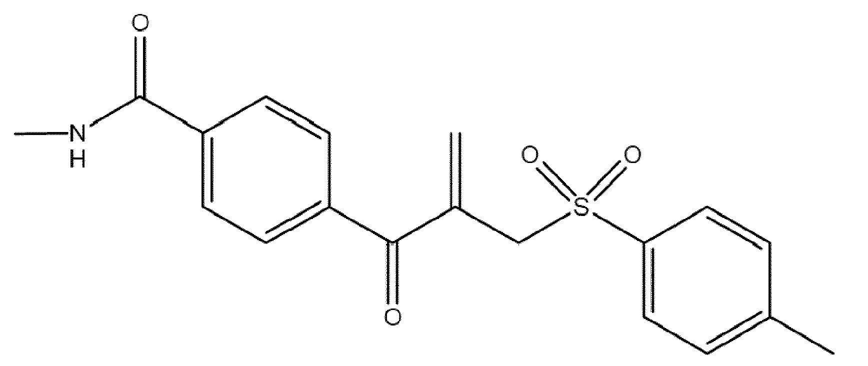
group, a 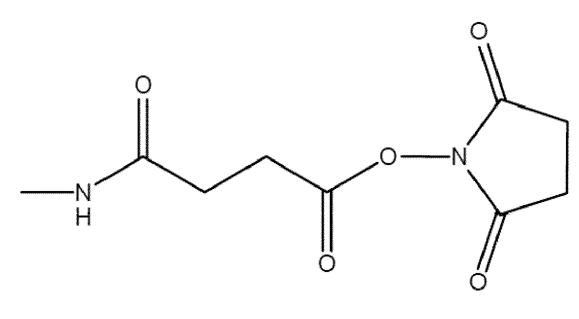 group or a 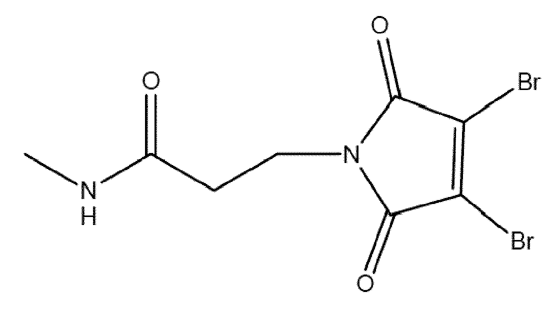 group,
comprising the steps of
(a) reacting a Paclitaxel or a Paclitaxel derivatives with the respective equivalents of a Fmoc amino protected linear amino alkanoic acid to obtain an 2'OH- and 7'OH- Fmoc di-protected Paclitaxel derivative, wherein the linear amino alkanoic acid is selected from the group consisting of glycine, aminopropionic acid, aminobutyric acid, aminovaleric acid, aminocapronic acid, aminoenanthic acid, aminocyprylic acid, aminopelargonic acid, aminocapric acid and aminoundecylic acid;
(b) cleaving of the 2'OH-Fmoc amino protected linear amino alkanoic acid and deprotecting of the 7'OH Fmoc protecting group by treatment with a base to obtain the linear amino alkanoic acid derivative of Paclitaxel or the Paxlitaxel derivates linked via the 7'OH of Paclitaxel or the Paclitaxel derivative,
(c) reacting the compound of step (b) with the reaction product of a trimeric acid linker having the structural elements of formula (I) or (II) as defined above, in order to obtain said Paclitaxel- or Paclitaxel derivatives linker-conjugate,

7. A Methotrexate-linker-conjugate-human serum albumin compound, wherein the chemical group Y of the Methotrexate-linker-conjugate according to claims 1 to 5 having formula (I) or (II), wherein D of R1 or R3 is Methotrexate, was chemically reacted with the thiol group of a Cysteine residue of human serum albumin or the amino group of a Lysine residue of human serum albumin.

8. A pharmaceutical composition comprising at least a conjugate according to any of the preceding claims or a pharmaceutically acceptable salt or solvate thereof and optionally pharmaceutically acceptable excipients, carriers or diluents.

9. A pharmaceutical kit comprising (i) at least a conjugate according to claims 1 to 5 or a pharmaceutical composition according to claim 8 and (ii) an anti-inflammatory, anti-viral or antiproliferative drug.

10. Conjugate, pharmaceutical composition or pharmaceutical kit according to any of the preceding claims for use as a medicament or a tool in biomedical research.

11. Conjugate, pharmaceutical composition or pharmaceutical kit according to any of the preceding claims for the treatment of a disease or disorder that can be at least in part alleviated by therapy.

12. Conjugate, pharmaceutical composition or pharmaceutical kit according to claim 11, wherein the disease or disorder is selected from the group consisting of inflammatory, auto-inflammatory or infectious disease or disorders, rheumatoid arthritis or cancer.
